# EUROPEAN PATENT SPECIFICATION

(11) **EP 2 928 567 B1**
(45) Date of publication and mention of the grant of the patent: **13.03.2019**
(21) Application number: 13814458.9
(22) Date of filing: 04.12.2013
(51) Int. Cl.: A61Q 3/02, A61K 8/37, A61K 8/73, A61K 8/35

(54) **NOVEL PHOTOCROSSLINKABLE COMPOSITIONS FOR USE AS BASE COAT**
NEUARTIGE LICHTVERNETZBARE ZUSAMMENSETZUNGEN ZUR VERWENDUNG ALS BASISLACK
NOUVELLES COMPOSITIONS PHOTORÉTICULABLES DESTINÉES À ÊTRE UTILISÉES COMME VERNIS DE BASE

(30) Priority: 05.12.2012 FR 1261674; 18.01.2013 US 201361754045 P
(43) Date of publication of application: 14.10.2015
(73) Proprietor: L'Oréal, 75008 Paris (FR)
(72) Inventor: KERGOSIEN, Guillaume, F-92370 Chaville (FR)
(74) Representative: Lavoix
(86) International application number: PCT/EP2013/075560
(87) International publication number: WO 2014/086877

(56) References cited:
- US-A1- 2011 060 065
- US-A1- 2011 081 306
- US-A1- 2011 082 228

## Description

The present invention relates to novel photocrosslinkable varnish compositions for use as a base coat. It also relates to the use of said compositions for nail and/or false nail makeup and/or treatment.

Nail varnish compositions may be used as a base for the varnish (or *base-coat*), as a nail makeup product, or as a finishing composition (or *top-coat*) to be applied on the nail makeup product, or as a cosmetic nail care product. These compositions may be applied onto natural nails as well as onto false nails.

In the field of nail varnishes, liquid cosmetic compositions are known, which are used by first applying a coat onto the nail and then subjecting said coat to the action of light radiation, inducing *in situ* polymerization and/or crosslinking reactions within said coat, resulting in generally crosslinked polymeric networks. Such photocrosslinkable compositions, commonly referred to as "UV gels" and generally based on (meth)acrylate monomer type crosslinkable compounds, are suitable for obtaining a satisfactory stability of the coat applied on the nail, and are described for example in CA 1 306 954, US 5 456 905, US 7 375 144 and FR 2 823 105, US 2011 0060065, US 2011 008228.

Some products substantially free from (meth)acrylate monomers are also on the market. However, these products pose performance problems particularly in respect of makeup quality and varnish stability over time.

The aim of the present invention is to provide novel photocrosslinkable compositions which do not have the drawbacks of the aforementioned alternative compositions.

In particular, the aim of the present invention is to provide photocrosslinkable compositions, preferably substantially free from (meth)acrylate monomers, which have a satisfactory stability in relation to the photocrosslinkable compositions currently available, notably those substantially free from (meth)acrylate monomers.

The aim of the present invention is to provide photocrosslinkable compositions, preferably substantially free from (meth)acrylate monomers, which are easy to use.

Another aim of the invention is to obtain photocrosslinkable compositions suitable for providing coats having the following properties: stability over time (with a mild etching or without any etching of the nail or false nail before applying the composition), easy makeup removal, high cosmeticity, outstanding makeup result (homogeneous deposition, easy to apply, comfortable to wear) and/or high gloss.

The present invention relates to a photocrosslinkable cosmetic composition as defined in the claims, comprising in a physiologically acceptable medium:
a) at least one photocrosslinkable compound comprising at least two (meth)acrylate functions and at least one carboxylic acid function;
b) at least one film-forming polymer in a proportion greater than or equal to 30% by weight in relation to the dry extract weight of the composition;
c) at least one volatile solvent in a proportion greater than or equal to 30% in relation to the total weight of the composition; and
d) at least one photoinitiator,
said composition comprising preferably less than 10% by weight of (meth)acrylate monomer in relation to the total weight of said composition.

According to one embodiment, the compositions according to the invention are photocrosslinkable compositions comprising a reduced quantity of (meth)acrylate monomer. In this way, they comprise preferably less than 5%, or less than 2%, or more preferentially less than 1%, by weight of (meth)acrylate monomer in relation to the total weight of said composition.

According to one advantageous embodiment, the compositions according to the invention are substantially free from (meth)acrylate monomer. Advantageously, the composition according to the invention is completely free from (meth)acrylate monomer.

The term "(meth)acrylate monomer" refers to a compound comprising a single (meth)acrylate function according to the formula H₂C=C(R)-C(O)-O-, where R = H or CH₃.

Within the scope of the present invention, compounds a), b), c) and d) above are separate entities.

### Physiologically acceptable medium

The cosmetic compositions according to the invention comprise a physiologically acceptable medium.

The term "physiologically acceptable medium" refers to a medium that is particularly suitable for the application of a composition of the invention onto keratin matter.

The physiologically acceptable medium is generally suitable for the nature of the support to which the composition should be applied, and also for the way in which the composition is to be packaged.

### Photocrosslinkable compound a)

The compositions according to the invention comprise at least one photocrosslinkable compound a). They may thus comprise a single photocrosslinkable compound or a mixture of a plurality of photocrosslinkable compounds.

According to one embodiment, the compositions according to the invention comprise a single photocrosslinkable compound as defined hereinafter.

Within the scope of the present invention, the term "photocrosslinkable compound" refers to an organic compound suitable for crosslinking under the action of a light ray, resulting in a crosslinked polymer network.

Compounds a) are defined by the presence of at least one carboxylic acid function, i.e. one -COOH function, and at least two (meth)acrylate functions, i.e. H₂C=C(R)-C(O)-O- functions, where R = H or CH₃.

According to one embodiment, compounds a) comprise at least two carboxylic acid functions and at least two (meth)acrylate, preferably methacrylate, functions.

According to one embodiment, compounds a) comprise at least two carboxylic acid functions and four (meth)acrylate functions, preferably four methacrylate functions.

According to one embodiment, compound a) is of formula (I): 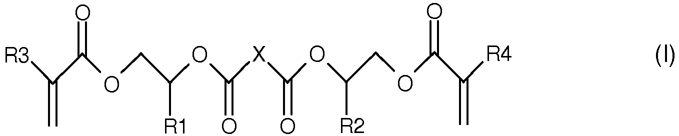 wherein:
- R1 and R2, identical or different, represent a hydrogen atom, or a methyl group, or a group of formula (II): 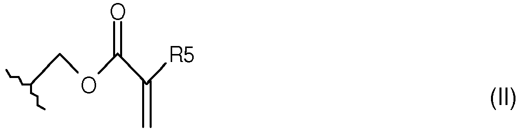 where R5 represents a hydrogen atom or a methyl group;
- R3 and R4, identical or different, represent a hydrogen atom or a methyl group;
- X represents a radical of any of the following formulae (III), (IV), (V), (VI), (VII), (VIII), (IX), or (X): 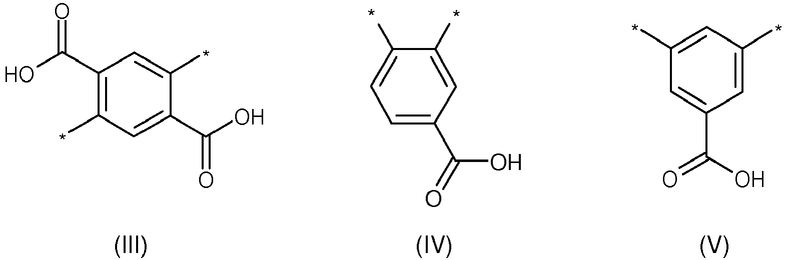 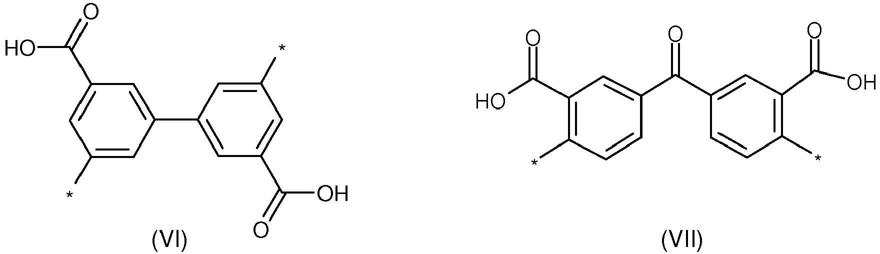 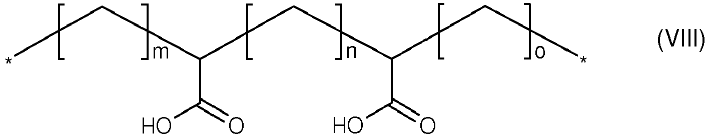 where m, n, and o, identical or different, represent an integer between 0 and 10, preferably between 0 and 1; 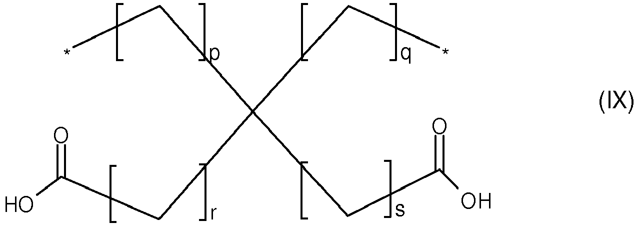 where p, q, r, and s, identical or different, represent an integer between 0 and 10, preferably between 0 and 1; 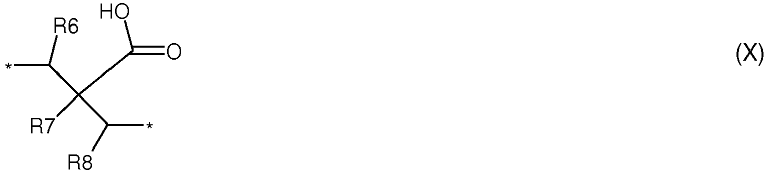 where R6, R7 and R8, identical or different, represent a hydrogen atom or a hydroxyl group.

In the above formulae, the * symbols represents the binding sites of the -X-radical.

According to one embodiment, compound a) complies with formula (I) as defined above, wherein the radical X is an aromatic radical, particularly an arylene radical, and preferably a phenylene radical.

Preferably, compound a) is of formula (I-1): 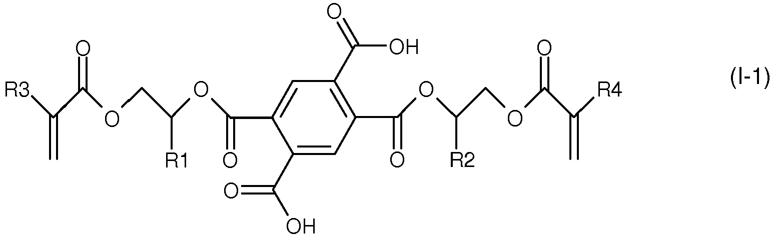 where R1, R2, R3 and R4 are as defined above in formula (I).

According to one embodiment, the compounds a) are of formula (I) or (I-1) as defined above, wherein R3 and R4 are methyl groups.

According to one embodiment, the compounds a) are of formula (I) or (I-1) as defined above, wherein R1 and R2, identical or different, represent a group of formula (II-1):

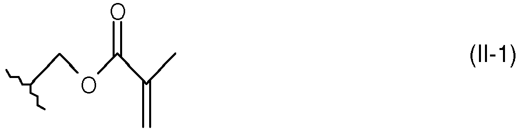

Preferentially, compound a) is of formula (I-2):

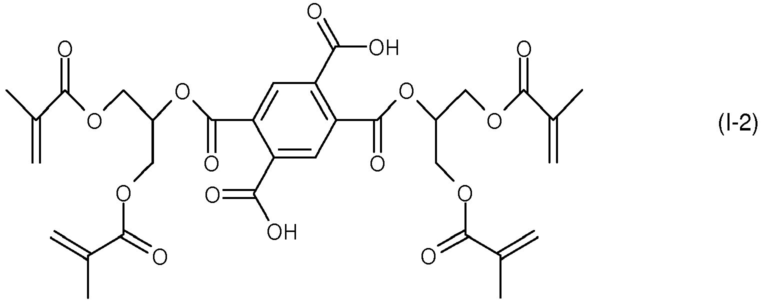

According to one embodiment, the compositions according to the invention have a compound a) content ranging from 1% to 10%, and preferably from 2% to 7%, by weight in relation to the total weight of the composition.

### Film-forming polymer b)

The compositions according to the invention also comprise at least one, or at least two, film-forming polymer(s).

In this way, the compositions according to the invention may comprise a single polymer b) or a mixture of a plurality of polymers b).

Preferably, the compositions according to the invention comprise a mixture of polymers b). According to this embodiment, they comprise at least two film-forming polymers b).

According to one advantageous embodiment, the compositions according to the invention comprise two polymers b).

The film-forming polymer content is at least 30% by weight in relation to the dry extract weight of the composition. Preferably, this content is greater than or equal to 40%, and preferably greater than or equal to 50%, by weight in relation to the dry extract weight of the composition.

The term "film-forming polymer" refers to, according to the invention, a polymer suitable for forming alone (i.e. in the absence of an auxiliary film-forming agent or an external stimulus for example such as UV), a film suitable for being isolated, particularly a continuous adherent film, on a substrate, particularly on nails.

The film-forming polymer is chosen from the group consisting of nitrocellulose, cellulose acetopropionate, cellulose acetobutyrate, and (meth)acrylate homopolymers and copolymers, and mixtures thereof.

Preferentially, in the compositions according to the invention, polymer b) is a mixture of nitrocellulose and a (meth)acrylate copolymer. Preferably, according to this embodiment, the ratio between the weight of nitrocellulose and the weight of (meth)acrylate copolymer is less than or equal to 1, and preferentially between 0.5 and 1.

### Volatile solvent c)

The compositions according to the invention comprise at least one volatile solvent c). They may thus comprise a single solvent c) or a mixture of a plurality of volatile solvents.

The mass volatile solvent content is preferably between 40% and 80%, preferably between 50% and 70%.

The term "volatile solvent" refers to a solvent capable of evaporating on contact with keratin matter, in less than one hour, at ambient temperature and at atmospheric pressure.

The volatile solvent(s) according to the invention are liquid solvents at ambient temperature, having a vapor pressure different to zero, at ambient temperature and atmospheric pressure, particularly ranging from 0.13 Pa to 40,000 Pa (from 10⁻³ to 300 mm Hg), particularly ranging from 1.3 Pa to 13,000 Pa (from 0.01 to 100 mm Hg), and more specifically ranging from 1.3 Pa to 1300 Pa (from 0.01 to 10 mm Hg).

Preferably, the solvents c) are chosen from polar solvents.

The term "polar" solvent, according to the present invention, refers to a solvent, or an oil, wherein the solubility parameter calculated over the melting point δₐ thereof is different to 0 (J/cm³)^{½}.

The definition and calculation of HANSEN three-dimensional solubility parameters are described in the article by C. M. HANSEN: "The three dimensional solubility parameters" J. Paint Technol. 39, 105 (1967).

According to the Hansen space:
- δ_{D} characterizes the LONDON dispersion forces derived from the formation of dipoles induced during molecular shocks;
- δₚ characterizes the DEBYE interaction forces between permanent dipoles and the KEESOM interaction forces between induced dipoles and permanent dipoles;
- δₕ characterizes the specific interaction forces (such as hydrogen, acid/base, donor/acceptor bonds, etc.); and
- δₐ is determined by the equation: δₐ= (δₚ² + δₕ²)^{½}.

The parameters δₚ, δₕ, δ_{D} et δₐ are expressed in (J/cm³)^{½}.

In particular, the term "polar" solvent refers to a solvent wherein the chemical structure is essentially formed from, or consists of, carbon and hydrogen atoms, and comprising at least one highly electronegative heteroatom such as an oxygen, nitrogen, silicon or phosphorus atom.

Preferably, this polar volatile solvent is chosen from the group consisting of C3-C6 esters and ketones and mixtures thereof.

As an example of a polar volatile solvent, mention may be made of acetone, methylethylketone, methylisobutylketone, cyclohexanone and alkyl acetates wherein the alkyl group comprises from 2 to 5 carbon atoms, such as methyl acetate, ethyl acetate, propyl acetate, n-propyl acetate, isopropyl acetate, n-butyl acetate, isobutyl acetate and tert-butyl acetate.

Preferably, the polar volatile solvent is C3-C5, and more preferentially chosen from the group consisting of ethyl acetate, n-propyl acetate, isopropyl acetate and mixtures thereof.

According to one preferred embodiment, solvent c) is a mixture of acetone, butyl acetate and ethyl acetate.

### Photoinitiator d)

The compositions according to the invention comprise at least one photoinitiator.

They may comprise a single photoinitiator or a mixture of a plurality of photoinitiators.

According to one embodiment, the compositions according to the invention comprise two photoinitiators.

The photoinitiators suitable for use according to the present invention are known in the art and are described, for example in "Les photoinitiateurs dans la reticulation des revêtements", G. Li Bassi, Double Liaison - Chimie des Peintures, No. 361, November 1985, p.34-41; "Applications industrielles de la polymerisation photoinduite", Henri Strub, L'Actualite Chimique, February 2000, p.5-13; and "Photopolymères: considerations théoriques et reaction de prise", Marc, J.M. Abadie, Double Liaison - Chimie des Peintures, No. 435-436, 1992, p.28-34.

These photoinitiators include:
- α-hydroxyketones, marketed for example under the names DAROCUR® 1173 and 4265, IRGACURE® 184, 2959, and 500 by BASF, and ADDITOL® CPK by CYTEC,
- α-aminoketones, marketed for example under the names IRGACURE® 907 and 369 by BASF,
- aromatic ketones marketed for example under the name ESACURE® TZT by LAMBERTI. Mention may also be made of thioxanthones marketed for example under the name ESACURE® ITX by LAMBERTI, and quinones. These aromatic ketones generally require the presence of a hydrogen donor compound such as tertiary amines and particularly alkanolamines. Mention may particularly be made of the tertiary amine ESACURE® EDB marketed by LAMBERTI.
- α-dicarbonyl derivatives of which the most common is benzyl dimethyl ketal marketed under the name IRGACURE® 651 by BASF. Further commercial products are marketed by LAMBERTI under the name ESACURE® KB1, and
- acylphosphine oxides, such as for example bis-acylphosphine oxides (BAPO) marketed for example under the names IRGACURE® 819, 1700, and 1800, DAROCUR® 4265, LUCIRIN® TPO, and LUCIRIN® TPO-L by BASF.

Preferably, the photoinitiator is chosen from the group consisting of α-hydroxyketones, α-aminoketones, aromatic ketones preferably associated with a hydrogen donor compound, aromatic α-diketones and acylphosphine oxides, and mixtures thereof.

A mixture of photoinitiators absorbing light radiation at various wavelengths is preferably used in the photocrosslinkable composition according to the invention. The absorption spectrum of the photocrosslinkable composition can thus be adapted to the emission spectrum of the light sources used.

Preferably, the composition according to the invention comprises a mixture of two different photoinitiators, such as for example a mixture of an α-hydroxyketone and an acylphosphine oxide.

As a photoinitiator mixture, mention may be made of a mixture of IRGACURE® 184 (BASF) and LUCIRIN® TPO-L (BASF).

A particular group of photoinitiators suitable for use in the photocrosslinkable cosmetic compositions according to the present invention is that of copolymerizable photoinitiators. It consists of molecules comprising both a photoinitiator group capable of photoinduced radical splitting and at least one double ethylene bond. The photoinitiators in this group offer the advantage, in relation to the conventional photoinitiators listed above, of being suitable for being incorporated, via the double bond, into the macromolecular system. This possibility reduces the content of free residual photoinitiators not having undergone photoinduced radical splitting and thus enhances the safety of the layer of varnish.

As examples of such copolymerizable photoinitiators, mention may be made of benzophenone acrylate derivatives marketed by CYTEC under the names EBECRYL® P36, EBECRYL® P37.

In one preferred embodiment of the invention, polymer photoinitiators or photoinitiators bound onto a high molar mass molecule are used. The choice of such a high mass photoinitiator offers the same advantage as selecting only polymeric copolymerizable compounds, i.e. enhanced safety of the photocrosslinkable cosmetic compositions due to the absence of very reactive molecules liable to diffuse to neighboring biological substrates. The mean molar mass by weight of the photoinitiator is preferably at least equal to 500 g/mol.

For example, mention may be made of an α-hydroxyketone oligomer corresponding to the following formula: 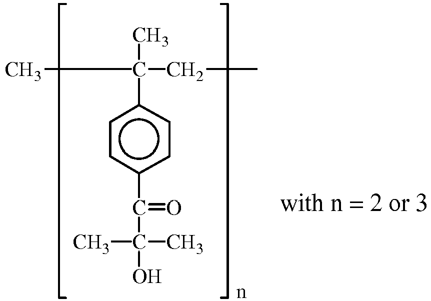 and which is marketed under the name ESACURE® KIP 150 by LAMBERTI.

The polymer on which the photoinitiator group is bound may optionally comprise one or a plurality of double ethylene bonds for optionally incorporating, into the macromolecular network, photoinitiator molecules not having undergone photoinduced splitting.

As examples of such high molar mass photoinitiators bearing double ethylene bonds, mention may be made of those corresponding to the following formulae: 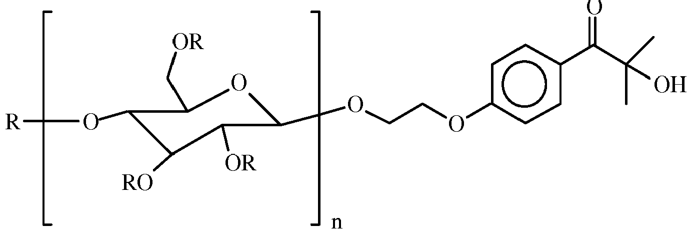 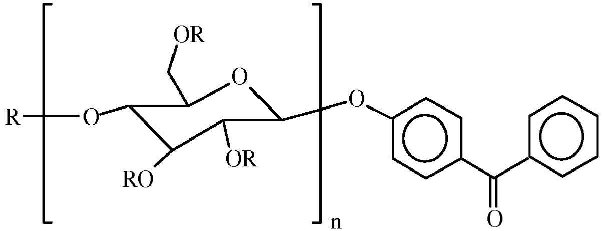 with n = 1 to 20 R = H or 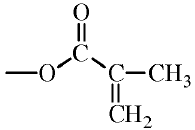 or

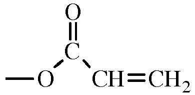

These structures are described in the following articles: S. Knaus, Pure Appl. Chem., A33(7), 869 (1996); S. Knaus, J. Polym. Sci, Part A = Polym. Chem., 33, 929 (1995); and R. Liska, Rad'Tech Europe 97, Lyon, F, 1997, Conference Proceedings.

The photoinitiator content is dependent on a large number of factors such as the reactivity of the various constituents of the mixture, the presence of pigments or dyes, the crosslinking density sought, the intensity of the light source or the exposure time.

In order to obtain satisfactory mechanical properties, the photoinitiator(s) is (or are) preferably present in a total content greater than or equal to 0.1% by weight in relation to the total weight of the photocrosslinkable composition, preferably ranging from 1 to 5% by weight in relation to the total weight of the photocrosslinkable composition.

### Other constituents

The compositions according to the invention may further comprise at least one photocrosslinkable urethane (meth)acrylate compound.

The term "urethane (meth)acrylate compound" refers to any compound comprising at least one urethane function -O-C(O)-NH-, and at least one (meth)acrylate function according to the formula H₂C=C(R)-C(O)-O-, where R = H or CH₃.

The "urethane" function is also referred to as a "carbamate" function.

As examples of urethane (meth)acrylate compounds, mention may be made of urethane poly(meth)acrylate compounds, particularly urethane di(meth)acrylate compounds, and more particularly urethane dimethacrylate compounds.

According to the present invention, the term "poly(meth)acrylate compound" refers to a (meth)acrylate compound comprising a plurality of (meth)acrylate functions.

In this way, the term "poly(meth)acrylate compound" may refer to a compound comprising at least two methacrylate functions, or at least two acrylate functions, or at least one methacrylate function and at least one acrylate function.

Advantageously, the mean number of (meth)acrylate functions borne by the photocrosslinkable urethane (meth)acrylate compound intended to form, after crosslinking, a crosslinked polymeric network, is greater than 1. Indeed, a polymerizable system consisting of molecules each bearing a single (meth)acrylate function forms, after reacting all of said functions, a linear or branched, and not crosslinked, chain macromolecular system. Only the presence of a certain fraction of molecules bearing at least two (meth)acrylate functions and thus acting as a crosslinking agent is suitable for obtaining a crosslinked polymeric system.

In the implementation of the present invention, the mean number of (meth)acrylate functions per molecule of urethane (meth)acrylate compound is preferably greater than or equal to 2, advantageously ranging from 2 to 6, preferably from 2 to 4, and more preferentially equal to 2.

Preferentially, this urethane (meth)acrylate compound is a urethane dimethacrylate compound. The term "urethane dimethacrylate compound" refers to any compound comprising at least one urethane function -O-C(O)-NH-, and two methacrylate functions according to the formula H₂C=C(CH₃)-C(O)-O-.

The photocrosslinkable compositions according to the invention may further comprise one or a plurality of plasticizers.

According to one embodiment, these compositions comprise less than 15% by weight of plasticizer in relation to the total weight of said composition.

Preferably, the mass plasticizer content ranges from 0% to 15%, preferentially from 1% to 10%, and more preferentially from 5% to 10%.

As examples of plasticizers, mention may particularly be made of standard plasticizers such as glycols and derivatives thereof, such as diethylene glycol ethylether, diethylene glycol methylether, diethylene glycol butylether or diethylene glycol hexylether, ethylene glycol ethylether, ethylene glycol methylether, ethylene glycol butylether, ethylene glycol hexylether, glycol esters, propylene glycol derivatives and particularly propylene glycol phenylether, propylene glycol diacetate, dipropylene glycol butylether, tripropylene glycol butylether, propylene glycol methylether, dipropylene glycol ethylether, tripropylene glycol methylether and diethylene glycol methylether, propylene glycol butylether, acid esters particularly carboxylic acid esters, such as citrates, particularly triethyl citrate, tributyl citrate, triethyl acetylcitrate, tributyl acetylcitrate, triethyl-2 hexyl acetylcitrate; phthalates, particularly dimethoxyethyl phthalate; phosphates, particularly tricresyl phosphate, tributyl phosphate, triphenyl phosphate, tributoxyethyl phosphate; tartrates, particularly dibutyl tartrate; adipates; carbonates; sebacates; benzyl benzoate, butyl acetyl ricinoleate, glyceryl acetyl ricinoleate, butyl glycolate, camphor, glycerol triacetate, N-ethyl-o,p-toluenesulfonamide, oxyethylenated derivatives such as oxyethylenated oils, particularly plant oils, such as castor oil, silicone oils, hydrocarbon oils, and mixtures thereof.

The composition according to the invention may further contain adjuvants, or additives, particularly chosen from pigments and dyes, coalescing agents, preservatives, waxes, thickeners, perfumes, UV filters, cosmetic active substances for nail care, spreading agents, anti-foaming agents and dispersing agents.

Obviously, those skilled in the art will take care to choose these optional adjuvants or additives such that the advantageous properties of the composition according to the invention are not, or are practically not, altered by the envisaged addition.

If the composition comprises pigments and/or dyes, it is particularly advisable to adapt the absorption spectrum of the pigments and/or dyes used to that of the photoinitiators, or conversely the absorption spectrum of the photoinitiators to that of the pigments and/or dyes used, so as to prevent both types of compounds from absorbing light at the same wavelengths. Indeed, the absorption of light by the pigments and/or dyes would render the photoinitiators present beyond a specific depth of the coat almost completely ineffective.

Preferably, the composition according to the invention is transparent.

As used herein, the term transparent refers to that the composition has a HAZEBYK index of less than 5 as measured with a KYKHAZEGLOSS type gloss meter.

According to one embodiment, the composition according to the invention further comprises a coloring agent chosen from the group consisting of soluble dyes, pigments, nacres and glitter.

The coloring agent(s) may be present in a total content greater than or equal to 0.1% by weight in relation to the total weight of the layer, ranging preferably from 0.1 to 5%, advantageously from 0.2 to 1% by weight in relation to the total weight of the composition.

The term "soluble dyes" should be understood to refer to organic, inorganic or organometallic compounds, soluble in the composition according to the invention and intended to color said composition.

The dyes are, for example, Sudan Red, DC Red 17, DC Green 6, β-carotene, soybean oil, Sudan brown, DC Yellow 11, DC Violet 2, DC Orange 5 and Quinoline Yellow.

The term "pigments" should be understood to refer to inorganic or organic, white or colored particles of any shape, insoluble in the composition according to the invention and intended to color said composition.

The term "nacres" should be understood to refer to iridescent particles of any shape, particularly produced by some mollusks in their shell or by synthetic means.

The pigments may be white or colored, inorganic and/or organic. Of the inorganic pigments, mention may be made of titanium dioxide, optionally surface-treated, zirconium or cerium oxides, along with zinc, iron (black, yellow or red) or chromium oxides, manganese violet, ultramarine blue, chromium hydrate and iron blue, metallic powders such as aluminum powder, copper powder.

Of the organic pigments, mention may be made of carbon black, D & C type pigments, and lacquers based on cochineal carmine, barium, strontium, calcium, aluminum.

Mention may also be made of effect pigments such as particles comprising a natural or synthetic organic or inorganic substrate, for example glass, acrylic resins, polyester, polyurethane, polyethylene terephthalate, ceramics, aluminas and optionally coated with metallic substances such as aluminum, gold, copper, bronze, or with metal oxides such as titanium dioxide, iron oxide, chromium oxide, inorganic or organic pigments and mixtures thereof.

The pearlescent pigments may be chosen from white pearlescent pigments such as mica coated with titanium, or bismuth oxychloride, colored pearlescent pigments such as titanium mica coated with iron oxides, titanium mica coated with iron blue and chromium oxide in particular, titanium mica coated with an organic pigments of the aforementioned type and pearlescent pigments based on bismuth oxychloride.

Pigments with goniochromatic properties may be used, particularly liquid crystal or multilayer pigments.

Optical brighteners or fibers optionally coated with optical brighteners may also be used.

The composition according to the invention may further comprise one or a plurality of fillers, particularly at a content ranging from 0.01% to 50% by weight, in relation to the total weight of the composition, preferably ranging from 0.01% to 30% by weight.

The term "fillers" should be understood to refer to inorganic or synthetic colorless or white particles of any shape, insoluble in the medium of the composition regardless of the temperature at which the composition is manufactured. These fillers may particularly be used to modify the rheology or texture of the composition.

The fillers may be mineral or organic particles of any shape, in sheet, spherical or oblong form, regardless of the crystallographic shape (for example sheet, cubic, hexagonal, orthorhombic, etc). Mention may be made of talc, mica, silica, kaolin, polyamide (Nylon®) (Orgasol® from Atochem), poly-β-alanine and polyethylene powders, tetrafluoroethylene polymer powders (Teflon®), lauroyl-lysine, starch, boron nitride, polymeric hollow microspheres such as those of polyvinylidene chloride/acrylonitrile like Expancel® (Nobel Industrie), acrylic acid copolymers (Polytrap® from Dow Corning) and silicone resin microbeads (Tospearls® from Toshiba, for example), elastomer polyorganosiloxane particles, precipitated calcium carbonate, magnesium carbonate and hydro-carbonate, hydroxyapatite, hollow silica microspheres (Silica Beads® from Maprecos), glass or ceramic microcapsules, metallic soaps derived from carboxylic organic acids having 8 to 22 carbon atoms, preferably from 12 to 18 carbon atoms, for example zinc, magnesium or lithium stearate, zinc laurate, magnesium myristate.

### Uses

According to one embodiment, the compositions according to the invention are intended to be applied onto nails and/or false nails.

In particular, the composition according to the invention is intended to be used as photocrosslinkable nail varnish.

Preferably, the photocrosslinkable cosmetic compositions are intended to be applied onto nails as a base coat.

The present invention also relates to a nail and/or false nail makeup and/or care method consisting of applying a photocrosslinkable composition as described above onto a nail and/or false nail.

As such, the present invention relates to a makeup and/or care method of a nail and/or false nail, comprising the following steps.
A) applying, onto a nail or a false nail, a photocrosslinkable composition according to the invention as defined above, whereby a coat consisting of at least one layer of said photocrosslinkable composition is deposited, and
B) exposing the coated nail or false nail obtained following step A) to UV or visible light radiation, whereby the photocrosslinking of the photocrosslinkable compound(s) of said composition is carried out.

The radiation suitable for the crosslinking of the photocrosslinkable composition according to the present invention has a wavelength between 210 and 600 nm, preferably between 250 and 420 nm, preferably between 350 and 410 nm. The use of lasers may also be envisaged.

In one preferred embodiment of the invention, a LED lamp or an UV lamp and particularly a mercury vapor lamp, optionally doped with further elements, such as gallium, suitable for modifying the emission spectrum of the light source, is used.

The exposure time of the deposited coat to radiation is dependent on various factors such as the chemical nature and content of the reactive compounds or the crosslinking density sought.

For nail varnishes, it would generally be sought to obtain satisfactory results for an exposure time between 10 seconds and 10 minutes, preferably between 30 seconds and 5 minutes.

Such a method may use a UV lamp having a power of approximately 36 W.

Preferably, the thickness after drying the coat of photocrosslinkable composition deposited in step A) is less than or equal to 100 µm, preferably less than or equal to 50 µm.

Following the final crosslinking step, the coat deposited on the nail or false nail may have a tacky layer on the surface thereof requiring cleaning of the crosslinked coat using for example a solvent such as isopropanol.

According to one embodiment, the method according to the invention further comprises, before step B), a period for drying the coat deposited following step A), the duration whereof may vary from 10 seconds to 10 minutes, typically from 30 seconds to 5 minutes. Said drying is generally performed in air and at ambient temperature.

A particular method according to the invention solely consists of a step A) and a step B) as defined above, optionally at an interval of a drying period as defined above.

According to one embodiment, after the crosslinking step B), the coat deposited on the nail is coated with at least one colored composition and/or a finishing composition, also known as a "*top-coat*", these compositions being optionally photocrosslinkable.

The crosslinked coat obtained from the crosslinking in step B) exhibits a significant stability over time, in terms of chipping resistance and gloss, particularly over the course of at least one week. It thus proves to be resistant to water, friction and shocks, and does not exhibit significant wear or chipping in this interval.

This coat is also capable of being solubilized or increasing in volume and thus weight when placed in contact with a standard makeup removal solvent. This ability to be solubilized or swell, displayed by the crosslinked coat, is specifically advantageous for the removal thereof when applied onto the surface of a nail or false nail. Indeed, the coat may be removed readily merely by means of makeup removal using a conventional solvent.

In this way, the composition according to the invention is advantageously suitable for being removed using standard solvents used in the field of nail varnish, and particularly using acetone and ethyl acetate, and mixtures thereof.

The present invention also relates to a nail and/or false nail makeup removal method, comprising the application of a makeup removal composition, such as a standard solvent described above, onto a nail or false nail coated with at least one layer obtained by crosslinking a layer of composition according to the invention, whereby said crosslinked layer is removed.

The present invention also relates to a kit comprising:
- a photocrosslinkable cosmetic composition according to the invention,
- an abrasive material having a granulometry greater than or equal to 200 µm, preferably less than 300 µm, advantageously comprised from 220 µm to 280 µm, and
- a LED lamp or an UV lamp.

The present invention also relates to a makeup and/or care method of a nail and/or false nail, comprising the following steps:
i) rubbing the surface of a nail or false nail with an abrasive material having a granulometry greater than or equal to 200 µm, preferably less than 300 µm, advantageously comprised from 220 µm to 280 µm,
ii) applying a photocrosslinkable composition according to the invention onto the surface of the nail or false nail which has been rubbed following step i), whereby a coat consisting of at least one layer of said photocrosslinkable composition is deposited, and
iii) exposing the coated nail or false nail obtained following step ii) to a LED lamp or an UV lamp, whereby the photocrosslinking of the photocrosslinkable composition is carried out.

Usually, the rubbing step is performed for less than 10 seconds, preferably less than 5 seconds, for example for approximately 3 seconds.

Throughout the application, the term "comprising a" or "including a" means "comprising at least one" or "including at least one", unless otherwise specified.

The weight percentages given in this application can be considered equivalent to the dry weight percentage of the compounds used.

The invention will be understood more clearly on reading the following description, given merely as an example.

### EXAMPLE

A layer of the base coat composition described below was applied onto nails.

After applying this layer, the film was crosslinked for 30 seconds under an "OPI GelColor" LED lamp from OPI.

| | |
|---|---|
| Pyromellitic dianhydride glycerol dimethacrylate (PMGDM) (X-830-0100 - ESSTECH, Inc.) | 5% |
| Urethane dimethacrylate (EXOTHANE 32 - ESSTECH, Inc.) | 5% |
| Nitrocellulose with 30% isopropyl alcohol (viscosity: E22 - 1/2s) | 12.244% |
| 50% acrylic copolymer in butyl acetate (PECOREZ AC 50 - PHOENIX CHEMICAL) | 22.86% |
| Acetone | 10% |
| Ethyl acetate | 26.526% |
| Butyl acetate | 15.3696% |
| Hydroxy Cyclohexylphenyl ketone photoinitiator (Irgacure 184 - BASF) | 2% |
| Ethyl-2,4,6-trimethylbenzoylphenylphosphinate photoinitiator (Lucirin TPO-L - BASF) | 1% |
| Alizurol purple SS (CI: 60725) (D&C VIOLET 2 - SENSIENT) | 0.0004% |

Two layers of the colored composition as described hereinafter were then applied.

After applying each of these two layers, the film was crosslinked for 30 seconds under an "OPI GelColor" LED lamp from OPI.

**Colored composition**

| | |
|---|---|
| Urethane dimethacrylate (EXOTHANE 10 - ESSTECH, Inc.) | 58.79% |
| Urethane dimethacrylate (EXOTHANE 32 - ESSTECH, Inc.) | 14.69% |
| 50% acrylic copolymer in butyl acetate (PECOREZ AC 50 - PHOENIX CHEMICAL) | 2% |
| Butyl acetate | 20% |
| Titanium oxide (PI-TAO-77891 - MIYOSHI KASEI) | 0.24% |
| Red 7 lacquer (Sunchroma D&C red 7 - Sun) | 0.7% |
| Red 6 lacquer (Sunchroma D&C red 6 - Sun) | 0.58% |
| Hydroxy Cyclohexylphenyl ketone photoinitiator (Irgacure 184 - BASF) | 2% |
| Ethyl-2,4,6-trimethylbenzoylphenylphosphinate photoinitiator (Lucirin TPO-L - BASF) | 1% |

One layer of the finishing composition as described hereinafter was then applied, and the film was crosslinked for 30 seconds under an "OPI GelColor" LED lamp from OPI.

After crosslinking the final layer, the surface is cleaned with cotton wool soaked in isopropanol.

**Finishing composition**

| | |
|---|---|
| Urethane dimethacrylate (EXOTHANE 10-ESSTECH, Inc.) | 58.4% |
| Urethane dimethacrylate (EXOTHANE 32 - ESSTECH, Inc.) | 14.6% |
| 50% acrylic copolymer in butyl acetate (PECOREZ AC 50 - PHOENIX CHEMICAL) | 2% |
| Butyl acetate | 19.9996% |
| Hydroxy Cyclohexylphenyl ketone photoinitiator (Irgacure 184 - BASF) | 2% |
| Ethyl- 2,4,6- trimethylbenzoylphenylphosphinate photo initiator (Lucirin TPO-L - BASF) | 3% |
| Alizurol purple SS (CI: 60725) (D&C VIOLET 2 - SENSIENT) | 0.0004% |

A varnish exhibiting satisfactory stability on the nail was thus obtained.

The varnish can be removed after placing in contact with acetone for 10 minutes.

## Claims

1. Photocrosslinkable cosmetic composition comprising in a physiologically acceptable medium:
a) at least one photocrosslinkable compound comprising at least two (meth)acrylate functions and at least one carboxylic acid function;
b) at least one film-forming polymer in a proportion greater than or equal to 30% by weight in relation to the dry extract weight of the composition, wherein said film-forming polymer is chosen from the group consisting of nitrocellulose, cellulose acetopropionate, cellulose acetobutyrate, and (meth)acrylate homopolymers and copolymers, and mixtures thereof;
c) at least one volatile solvent in a proportion greater than or equal to 30% in relation to the total weight of the composition; and
d) at least one photoinitiator.

2. Composition according to claim 1, wherein compound a) is of formula (I): 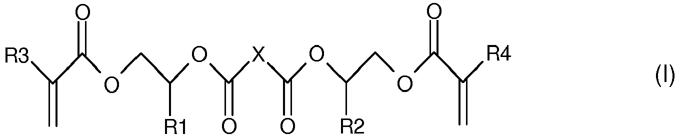 wherein:
- R1 and R2, identical or different, represent a hydrogen atom, or a methyl group, or a group of formula (II): 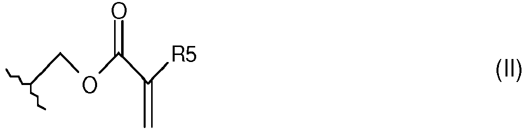 where R5 represents a hydrogen atom or a methyl group;
- R3 and R4, identical or different, represent a hydrogen atom or a methyl group;
- X represents a radical of any of the following formulae (III), (IV), (V), (VI), (VII), (VIII), (IX), or (X): 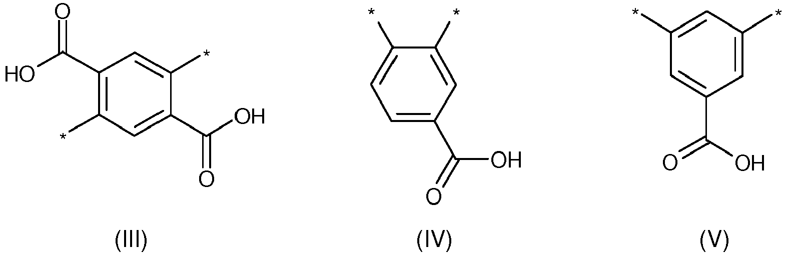 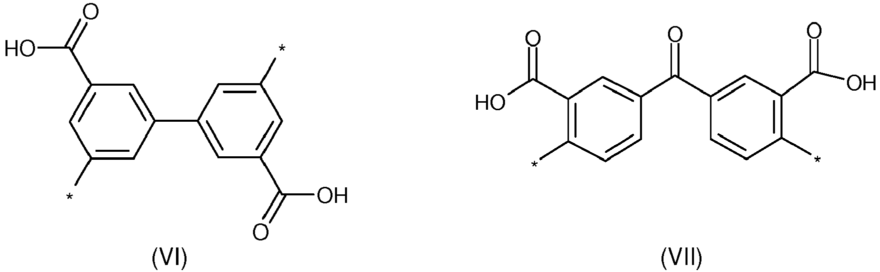 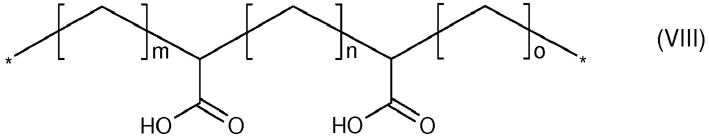 where m, n, and o, identical or different, represent an integer between 0 and 10; 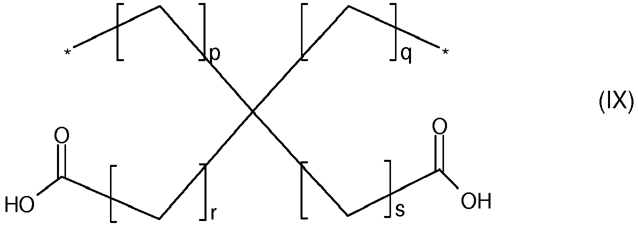 where p, q, r and s, identical or different, represent an integer between 0 and 10; 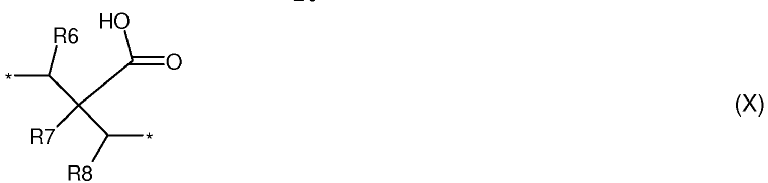
where R6, R7 and R8, identical or different, represent a hydrogen atom or a hydroxyl group.

3. Composition according to claim 2, wherein compound a) is of formula (I-1): 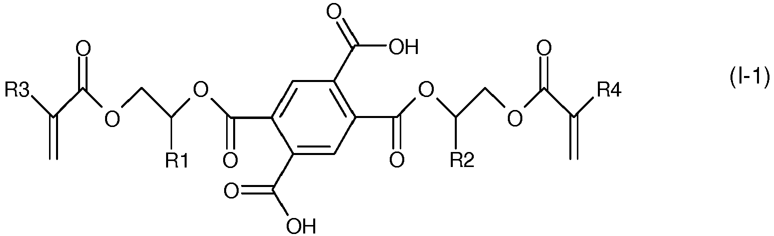 where R1, R2, R3 and R4 are as defined in claim 2;

4. Composition according to claim 3, wherein R3 and R4 are methyl groups.

5. Composition according to claim 3 or 4, wherein R1 and R2, identical or different, represent a group of formula (II-1):

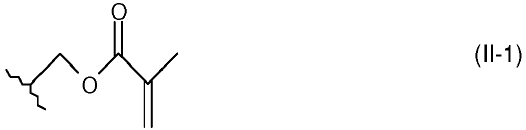

6. Composition according to any of claims 1 to 5, comprising less than 5% by weight of (meth)acrylate monomer in relation to the total weight of said composition.

7. Composition according to any of claims 1 to 6, comprising at least two film-forming polymers b).

8. Composition according to any of claims 1 to 7, wherein the polymer b) is a mixture of nitrocellulose and a (meth)acrylate copolymer.

9. Composition according to any of claims 1 to 8, wherein the volatile solvent is a polar volatile solvent chosen from the group consisting of C3-C6 esters and ketones and mixtures thereof.

10. Composition according to any of claims 1 to 9, wherein the photoinitiator is chosen from the group consisting of α-hydroxyketones, α-aminoketones, aromatic ketones preferably associated with a hydrogen donor compound, aromatic α-diketones, and acylphosphine oxides, and mixtures thereof.

11. Composition according to any of claims 1 to 10, further comprising at least one photocrosslinkable urethane (meth)acrylate compound.

12. Composition according to any of claims 1 to 11, further comprising at least one plasticizer.

13. Composition according to any of claims 1 to 12, **characterized in that** it is transparent.

14. Makeup and/or care method of a nail and/or false nail, comprising the following steps:
A) applying, onto a nail or a false nail, a composition according to any of claims 1 to 13, whereby a coat consisting of at least one layer of said photocrosslinkable composition is deposited, and
B) exposing the coated nail or false nail obtained following step A) to UV or visible light radiation.

15. Kit comprising:
- a photocrosslinkable cosmetic composition according to any one of claims 1 to 13,
- an abrasive material having a granulometry greater than or equal to 200 µm, preferably less than 300 µm, advantageously comprised from 220 µm to 280 µm, and
- a LED lamp or an UV lamp.

16. Makeup and/or care method of a nail and/or false nail, comprising the following steps:
i) rubbing the surface of a nail or false nail with an abrasive material having a granulometry greater than or equal to 200 µm, preferably less than 300 µm, advantageously comprised from 220 µm to 280 µm,
ii) applying a photocrosslinkable composition according to any one of claims 1 to 13 onto the surface of the nail or false nail which has been rubbed following step i), whereby a coat consisting of at least one layer of said photocrosslinkable composition is deposited, and
iii) exposing the coated nail or false nail obtained following step ii) to a LED lamp or an UV lamp, whereby the photocrosslinking of the photocrosslinkable composition is carried out.

## Patentansprüche

1. Photovernetzbare kosmetische Zusammensetzung umfassend in einem physiologisch akzeptablem Medium:
a) mindestens eine photovernetzbare Verbindung umfassend mindestens zwei (Meth)acrylat-Funktionen und mindestens eine Carbonsäure-Fu nktion;
b) mindestens ein filmbildendes Polymer in einem Anteil größer als oder gleich 30 Gew.-% bezogen auf das Trockenextraktgewicht der Zusammensetzung, wobei das filmbildende Polymer ausgewählt ist aus der Gruppe bestehend aus Nitrocellulose, Cellulose, Acetopropionat, Cellulose-Acetobutyrat, und (Meth)acrylat-Homopolymeren und -Copolymeren, und Mischungen davon;
c) mindestens ein leichtflüchtiges Lösungsmittel in einem Anteil größer als oder gleich 30 % bezogen auf das Gesamtgewicht der Zusammensetzung; und
d) mindestens einen Photoinitiator.

2. Zusammensetzung gemäß Anspruch 1, wobei Verbindung a) die Formel (I) aufweist: 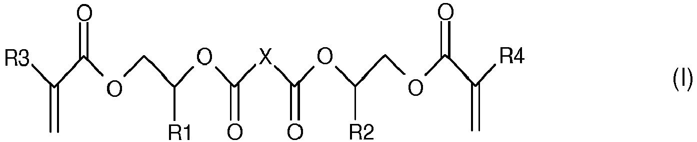 wobei:
- R1 und R2, identisch oder verschieden, ein Wasserstoffatom oder eine Methylgruppe oder eine Gruppe der Formel (II) darstellen: 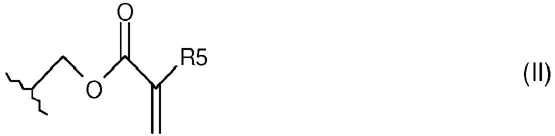 wobei R5 ein Wasserstoffatom oder eine Methylgruppe darstellt;
- R3 und R4, identisch oder verschieden, ein Wasserstoffatom oder eine Methylgruppe darstellen;
- X einen Rest einer der folgenden Formeln (III), (IV), (V), (VI), (VII), (VIII), (IX) oder (X) darstellt: 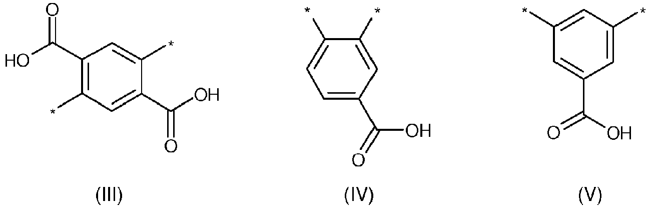 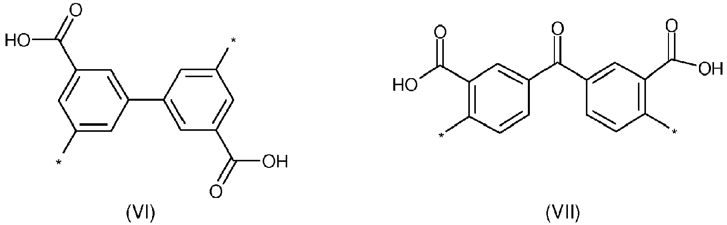 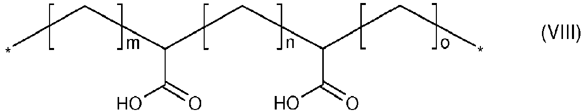 wobei m, n und o, identisch oder verschieden, eine ganze Zahl zwischen 0 und 10 darstellen; 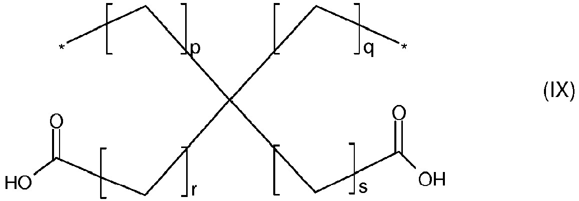 wobei p, q, r und s, identisch oder verschieden, eine ganze Zahl zwischen 0 und 10 darstellen; 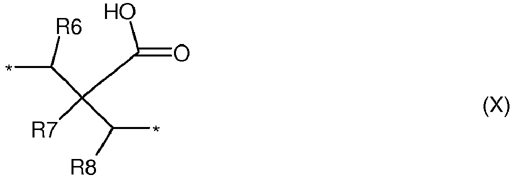 wobei R6, R7 und R8, identisch oder verschieden, ein Wasserstoffatom oder eine Hydroxylgruppe darstellen.

3. Zusammensetzung gemäß Anspruch 2, wobei Verbindung a) die Formel (I-1) aufweist: 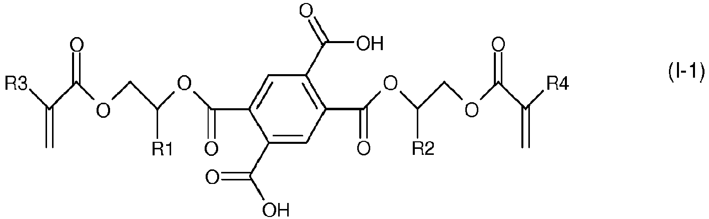 wobei R1, R2, R3 und R4 wie in Anspruch 2 definiert sind;

4. Zusammensetzung gemäß Anspruch 3, wobei R3 und R4 Methylgruppen sind.

5. Zusammensetzung gemäß Anspruch 3 oder 4, wobei R1 und R2, identisch oder verschieden, eine Gruppe der Formel (II-1) darstellen:

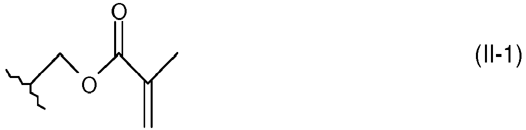

6. Zusammensetzung gemäß einem der Ansprüche 1 bis 5, umfassend weniger als 5 Gew.-% (Meth)acrylat-Monomer bezogen auf das Gesamtgewicht der Zusammensetzung.

7. Zusammensetzung gemäß einem der Ansprüche 1 bis 6, umfassend mindestens zwei filmbildende Polymere b).

8. Zusammensetzung gemäß einem der Ansprüche 1 bis 7, wobei das Polymer b) eine Mischung aus Nitrocellulose und einem (Meth)acrylat-Copolymer ist.

9. Zusammensetzung gemäß einem der Ansprüche 1 bis 8, wobei das leichtflüchtige Lösungsmittel ein polares leichtflüchtiges Lösungsmittel ausgewählt aus der Gruppe bestehend aus C3-C6-Estern und Ketonen und Mischungen davon ist.

10. Zusammensetzung gemäß einem der Ansprüche 1 bis 9, wobei der Photoinitiator ausgewählt ist aus der Gruppe bestehend aus α-Hydroxyketonen, α-Aminoketonen, aromatischen Ketonen, die bevorzugt mit einer Wasserstoff-Donator-Verbindung verbunden sind, aromatischen α-Diketonen, und Acylphosphinoxiden, und Mischungen davon.

11. Zusammensetzung gemäß einem der Ansprüche 1 bis 10, ferner umfassend mindestens eine photovernetzbare Urethan-(meth)acrylat-Verbindung.

12. Zusammensetzung gemäß einem der Ansprüche 1 bis 11, ferner umfassend mindestens einen Weichmacher.

13. Zusammensetzung gemäß einem der Ansprüche 1 bis 12, **dadurch gekennzeichnet, dass** sie transparent ist.

14. Make-up- und/oder Pflegeverfahren eines Nagels und/oder falschen Nagels, umfassend die folgenden Schritte:
A) Auftragen einer Zusammensetzung gemäß einem der Ansprüche 1 bis 13 auf einen Nagel oder einen falschen Nagel, wobei ein Film bestehend aus mindestens einer Schicht der photovernetzbaren Zusammensetzung aufgetragen wird, und
B) Aussetzen des nach Schritt A) erhaltenen beschichteten Nagels oder falschen Nagels gegenüber UV- oder sichtbarer Lichtstrahlung.

15. Kit umfassend:
- eine photovernetzbare kosmetische Zusammensetzung gemäß einem der Ansprüche 1 bis 13,
- ein abrasives Material, welches eine Granulometrie größer als oder gleich 200 µm, bevorzugt weniger als 300 µm, zweckmäßigerweise enthalten von 200 µm bis 280 µm, aufweist, und
- eine LED-Lampe oder eine UV-Lampe.

16. Make-up- und/oder Pflegeverfahren eines Nagels und/oder falschen Nagels, umfassend die folgenden Schritte:
i) Reiben der Oberfläche eines Nagels oder falschen Nagels mit einem abrasiven Material, welches eine Granulometrie größer als oder gleich 200 µm, bevorzugt weniger als 300 µm, zweckmäßigerweise enthalten von 200 µm bis 280 µm, aufweist,
ii) Auftragen einer photovernetzbaren Zusammensetzung gemäß einem der Ansprüche 1 bis 13 auf die Oberfläche eines nach Schritt i) geriebenen Nagels oder falschen Nagels, wobei ein Film bestehend aus mindestens einer Schicht der photovernetzbaren Zusammensetzung aufgetragen wird, und
iii) Aussetzen des nach Schritt ii) erhaltenen beschichteten Nagels oder falschen Nagels gegenüber einer LED-Lampe oder einer UV-Lampe, wobei die Photovernetzung der photovernetzbaren Zusammensetzung durchgeführt wird.

## Revendications

1. Composition cosmétique photoréticulable comprenant, dans un milieu physiologiquement acceptable :
a) au moins un composé photoréticulable comprenant au moins deux fonctions (méth)acrylate et au moins une fonction acide carboxylique ;
b) au moins un polymère filmogène en une proportion supérieure ou égale à 30 % en poids par rapport au poids de l'extrait sec de la composition, lequel polymère filmogène est choisi dans l'ensemble constitué par la nitrocellulose, l'acétopropionate de cellulose, l'acétobutyrate de cellulose et les homopolymères et copolymères de (méth)acrylate, ainsi que leurs mélanges ;
c) au moins un solvant volatil en une proportion supérieure ou égale à 30 % par rapport au poids total de la composition ; et
d) au moins un photoamorceur.

2. Composition selon la revendication 1, dans laquelle le composé a) est de formule (I) : 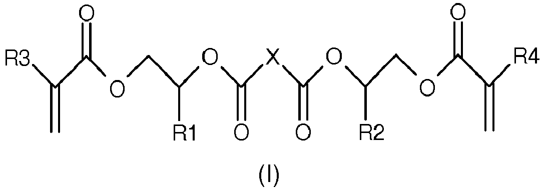 dans laquelle :
- R1 et R2, identiques ou différents, représentent un atome d'hydrogène, ou un groupe méthyle, ou un groupe de formule (II) : 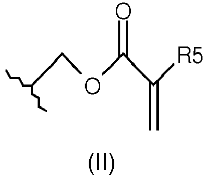 dans laquelle R5 représente un atome d'hydrogène ou un groupe méthyle ;
- R3 et R4, identiques ou différents, représentent un atome d'hydrogène ou un groupe méthyle ;
- X représente un radical de l'une quelconque des formules (III), (IV), (V), (VI), (VII), (VIII), (IX) et (X) suivantes : 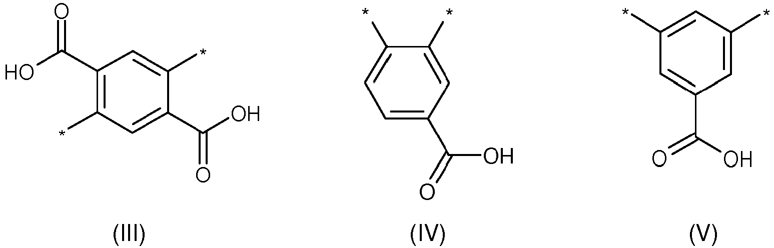 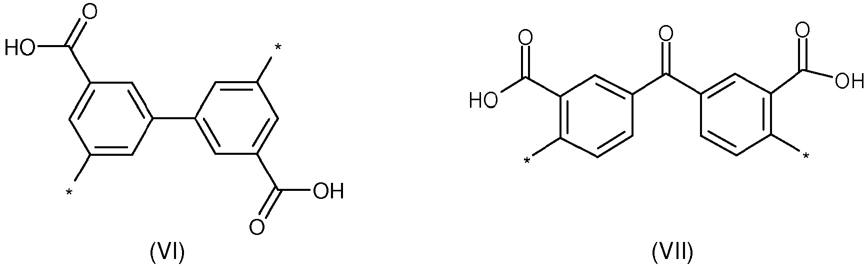 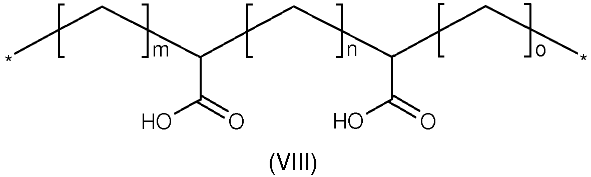 dans laquelle m, n et o, identiques ou différents, représentent un entier compris entre 0 et 10; 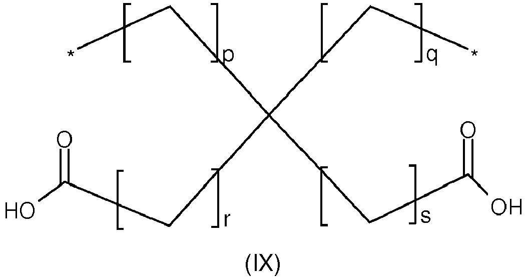 dans laquelle p, q, r et s, identiques ou différents, représentent un entier compris entre 0 et 10 ; 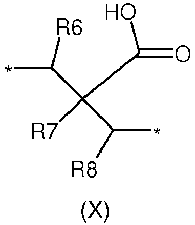 dans laquelle R6, R7 et R8, identiques ou différents, représentent un atome d'hydrogène ou un groupe hydroxyle.

3. Composition selon la revendication 2, dans laquelle le composé a) est de formule (I-1) : 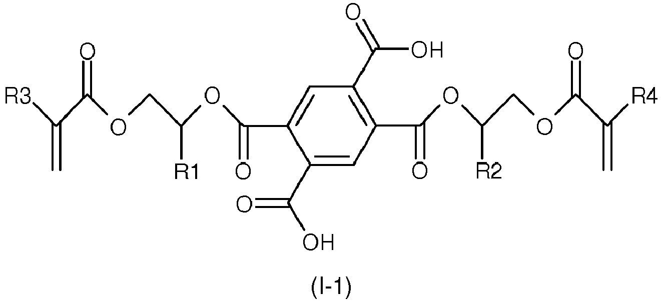 dans laquelle R1, R2, R3 et R4 sont tels que définis dans la revendication 2.

4. Composition selon la revendication 3, dans laquelle R3 et R4 sont des groupes méthyle.

5. Composition selon la revendication 3 ou 4, dans laquelle R1 et R2, identiques ou différents, représentent un groupe de formule (II-1) :

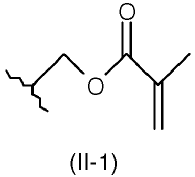

6. Composition selon l'une quelconque des revendications 1 à 5, comprenant moins de 5 % en poids de monomère de (méth)acrylate par rapport au poids total de ladite composition.

7. Composition selon l'une quelconque des revendications 1 à 6, comprenant au moins deux polymères filmogènes b).

8. Composition selon l'une quelconque des revendications 1 à 7, dans laquelle le polymère b) est un mélange de nitrocellulose et d'un copolymère de (méth)acrylate.

9. Composition selon l'une quelconque des revendications 1 à 8, dans laquelle le solvant volatil est un solvant volatil polaire choisi dans l'ensemble constitué par les cétones et les esters en C3 à C6, ainsi que leurs mélanges.

10. Composition selon l'une quelconque des revendications 1 à 9, dans laquelle le photoamorceur est choisi dans l'ensemble constitué par les α-hydroxycétones, les α-aminocétones, les cétones aromatiques de préférence associées à un composé donneur d'hydrogène, les α-dicétones aromatiques, et les oxydes d'acylphosphine, ainsi que leurs mélanges.

11. Composition selon l'une quelconque des revendications 1 à 10, comprenant en outre au moins un composé (méth)acrylate d'uréthane photoréticulable.

12. Composition selon l'une quelconque des revendications 1 à 11, comprenant en outre au moins un plastifiant.

13. Composition selon l'une quelconque des revendications 1 à 12, **caractérisée en ce qu'**elle est transparente.

14. Procédé de maquillage et/ou de soin d'un ongle et/ou d'un faux ongle, comprenant les étapes suivantes :
A) application, sur un ongle ou un faux ongle, d'une composition selon l'une quelconque des revendications 1 à 13, en conséquence de quoi un revêtement constitué d'au moins une couche de ladite composition photoréticulable est déposé, et
B) exposition de l'ongle ou du faux ongle revêtu obtenu suivant l'étape A) à un rayonnement de lumière visible ou UV.

15. Trousse comprenant :
- une composition cosmétique photoréticulable selon l'une quelconque des revendications 1 à 13,
- un matériau abrasif ayant une granulométrie supérieure ou égale à 200 µm, de préférence inférieure à 300 µm, avantageusement comprise entre 220 µm et 280 µm, et
- une lampe à LED ou une lampe à UV.

16. Procédé de maquillage et/ou de soin d'un ongle et/ou d'un faux ongle, comprenant les étapes suivantes :
i) frottement de la surface d'un ongle ou d'un faux ongle avec un matériau abrasif ayant une granulométrie supérieure ou égale à 200 µm, de préférence inférieure à 300 µm, avantageusement comprise entre 220 µm et 280 µm,
ii) application d'une composition photoréticulable selon l'une quelconque des revendications 1 à 13 sur la surface de l'ongle ou du faux ongle qui a été frottée suivant l'étape i), en conséquence de quoi un revêtement constitué d'au moins une couche de ladite composition photoréticulable est déposé, et
iii) exposition de l'ongle ou du faux ongle revêtu obtenu suivant l'étape ii) à une lampe à LED ou une lampe à UV, en conséquence de quoi la photoréticulation de la composition photoréticulable est effectuée.
